# EUROPEAN PATENT APPLICATION

(11) **EP 1 884 194 A1**
(43) Date of publication of application: **06.02.2008**
(21) Application number: 07013609.8
(22) Date of filing: 11.07.2007
(51) Int. Cl.: A61B 6/14

(54) **Dental radiation image display apparatus**

(30) Priority: 31.07.2006 JP 2006207548
(71) Applicant: FUJIFILM Corporation, Minato-ku Tokyo (JP)
(72) Inventor: Yasuda, Hiroaki, Ashigarakami-gun Kanagawa 258-8538 (JP); Yamazaki, Atsushi, Ashigarakami-gun Kanagawa 258-8538 (JP)
(74) Representative: Klunker . Schmitt-Nilson . Hirsch

(57) **Abstract**

A plurality of segmental images (66a through 66j) captured of teeth are analyzed, and feature information including directions in which the segmental images are oriented, angles of inclination of straight lines related to the teeth, and width and lengths of the teeth is extracted. According to the feature information, which may also include therapeutic information of the teeth, display positions and display postures of the segmental images (66a through 66j) are calculated, and the segmental images (66a through 66j) are displayed in locations based on the calculated display positions and display postures.

## Description

### BACKGROUND OF THE INVENTION

### Field of the Invention:

The present invention relates to a dental radiation image display apparatus for displaying a plurality of segmental images, which are set in respective predetermined positions, of teeth of a subject which are imaged by a radiation image capturing apparatus.

### Description of the Related Art:

For treating teeth, it has been customary for the dentist to capture a panoramic image as a radiation image of overall teeth or a segmental image thereof, interpret the captured image to grasp the state of the teeth, and determine a therapeutic strategy for the treatment of the teeth. General dental radiographic processes include a 10-film process for dividing the image of upper and lower teeth into segmental images on 10 films and a 14-film process for dividing the image of upper and lower teeth into segmental images on 14 films.

When a plurality of segmental images of teeth are recorded on X-ray films for interpretation by the dentist, it is mandatory to place the segmental images in respective positions according to the array of the teeth for letting the dentist interpret and diagnose the teeth properly.

It has been known to acquire digital radiation images from a CT image capturing apparatus and a radiation image capturing apparatus employing a stimulable phosphor panel. The digital radiation images acquired from these apparatus are displayed on a display device for interpretation and diagnosis.

When segmental digital images of teeth are displayed on a display device, they need to be displayed in respective positions according to the array of the teeth. According to the related art disclosed in Japanese laid-open patent publication No. 2001-333898, as shown in FIG. 5 of the accompanying drawings, a panoramic image 4 of teeth captured by a CT image capturing apparatus is displayed centrally, and segmental images 2a through 2j are displayed in respective positions above and below the panoramic image 4 according to the array of the teeth.

If the images are captured in a sequence according to the array of the teeth and their image information is supplied according to the same sequence to the display device, then the images are displayed according to the array of the teeth.

However, there is no guarantee that the operator who handles the image capturing apparatus will capture the images in the sequence according to the array of the teeth. In addition, the operator may possibly commit an error in capturing the images in the sequence according to the array of the teeth. Therefore, the operator or the dentist has to display the captured segmental images on the display device, confirm the positions of the displayed segmental images, and, if necessary, correct the positions of any misplaced segmental images. The confirming and correcting practice requires the operator or the dentist to be sufficiently experienced and skilled to be able to distinguish features of the segmental images.

### SUMMARY OF THE INVENTION

It is an object of the present invention to provide a dental radiation image display apparatus which is capable of automatically arranging and displaying a plurality of segmental images of teeth in positions suitable for interpretation regardless of the sequence in which the segmental images have been captured.

The above and other objects, features, and advantages of the present invention will become more apparent from the following description when taken in conjunction with the accompanying drawings in which a preferred embodiment of the present invention is shown by way of illustrative example.

### BRIEF DESCRIPTION OF THE DRAWINGS

FIG. 1 is a view showing a radiation image information processing system according to an embodiment of the present invention;
FIG. 2 is a functional block diagram of an image reading apparatus and a dental radiation image display apparatus of the radiation image information processing system shown in FIG. 1;
FIG. 3 is a flowchart of a processing sequence of the radiation image information processing system shown in FIG. 1;
FIG. 4 is a view showing a displayed image including segmental images of teeth captured according to a 10-film process and arranged according to an array of teeth, the displayed image being displayed by the dental radiation image display apparatus; and
FIG. 5 is a view showing images of teeth displayed according to related art.

### DESCRIPTION OF THE PREFERRED EMBODIMENT

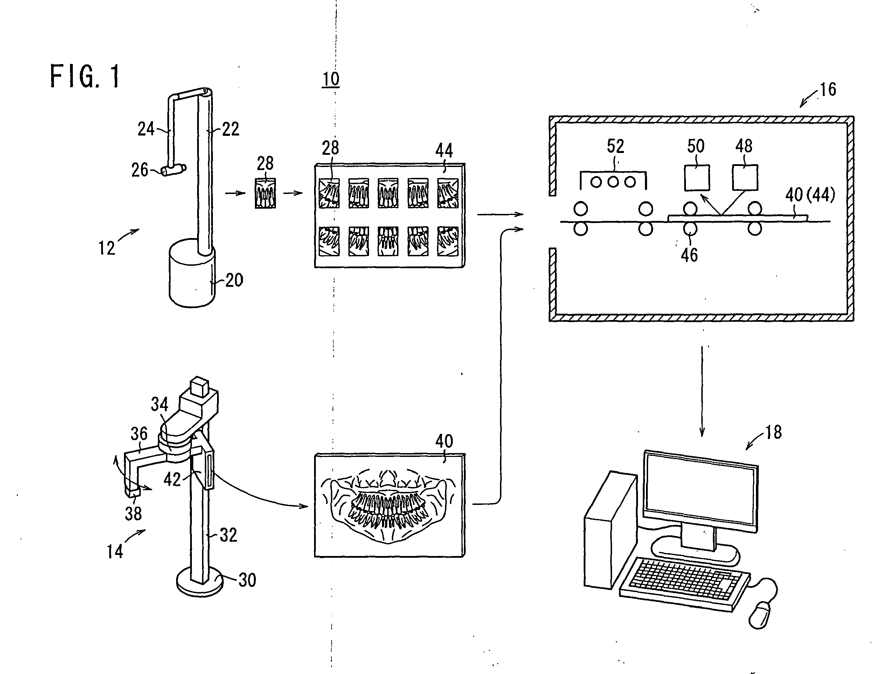

FIG. 1 shows a radiation image information processing system 10 according to an embodiment of the present invention. As shown in FIG. 1, the radiation image information processing system 10 comprises a segmental image capturing apparatus 12 for capturing segmental images of teeth of a subject, a panoramic image capturing apparatus 14 for capturing a panoramic image of all the teeth, an image reading apparatus 16 for reading radiation image information of the segmental images and the panoramic image to a recording medium, and a dental radiation image display apparatus 18 according to an embodiment of the present invention for arranging and displaying the segmental images and the panoramic image in respective predetermined positions.

The segmental image capturing apparatus 12 includes a universal arm 24 having an upper end coupled to the upper end of a vertical post 22 fixed to a base 20, and an imaging head 26 coupled to the lower end of the universal arm 24 and housing a radiation source. The segmental image capturing apparatus 12 records a segmental image of teeth on a stimulable phosphor panel 28 that serves as a recording medium.

The stimulable phosphor panel 28 is of a size commensurate with the range of a segmental image to be recorded thereon. For recording a segmental image of teeth on the stimulable phosphor panel 28, the stimulable phosphor panel 28 is held against the teeth by a finger or the like. The stimulable phosphor panel 28 comprises a support base and a stimulable phosphor layer disposed on the support base for storing the energy of a radiation applied thereto. When the stimulable phosphor panel 28 which has stored the energy of the applied radiation is irradiated with stimulating light, the stimulable phosphor panel 28 emits stimulated light. When the stimulable phosphor panel 28 is subsequently exposed to a certain amount of erasing light, the stimulable phosphor panel 28 discharges any remaining radiation energy and can be used again.

The panoramic image capturing apparatus 14 includes a turning mechanism 34 coupled to the upper end of a vertical post 32 fixed to a base 30, and an imaging head 38 fixed to an end of a swing arm 36 swingable by the turning mechanism 34 and housing a radiation source. An imaging base 42 which accommodates a long stimulable phosphor panel 40 as a recording medium therein is secured to the other end of the swing arm 36. The panoramic image capturing apparatus 14 records a panoramic image of teeth of a subject on the long stimulable phosphor panel 40 when the turning mechanism 34 turns the imaging head 38 and the imaging base 42 around the jaws of the subject.

The image reading apparatus 16 serves to read radiation image information from the stimulable phosphor panel 28 which has recorded segmental images and the stimulable phosphor panel 40 which has recorded a panoramic image. The stimulable phosphor panel 28 is smaller in size than the stimulable phosphor panel 40. When the stimulable phosphor panel 28 is supplied to the image reading apparatus 16, a plurality of stimulable phosphor panels 28 are mounted on an adapter 44, and the adapter 44 is supplied to the image reading apparatus 16.

The image reading apparatus 16 comprises a feed mechanism 46 for feeding the adapter 44 on which the stimulable phosphor panels 28 are mounted and or the stimulable phosphor panel 40, a scanner 48 for scanning the stimulable phosphor panels 28 or the stimulable phosphor panel 40 with stimulating light, a reader 50 for collecting stimulating light that is emitted from the stimulable phosphor panel 28 or the stimulable phosphor panel 40 upon exposure to the stimulating light, and converting the collected stimulating light into an electric signal, and an eraser 52 for erasing remaining radiation image information from the stimulable phosphor panel 28 or the stimulable phosphor panel 40 by applying erasing light to the stimulable phosphor panel 28 or the stimulable phosphor panel 40 from which the desired radiation image information has been read.

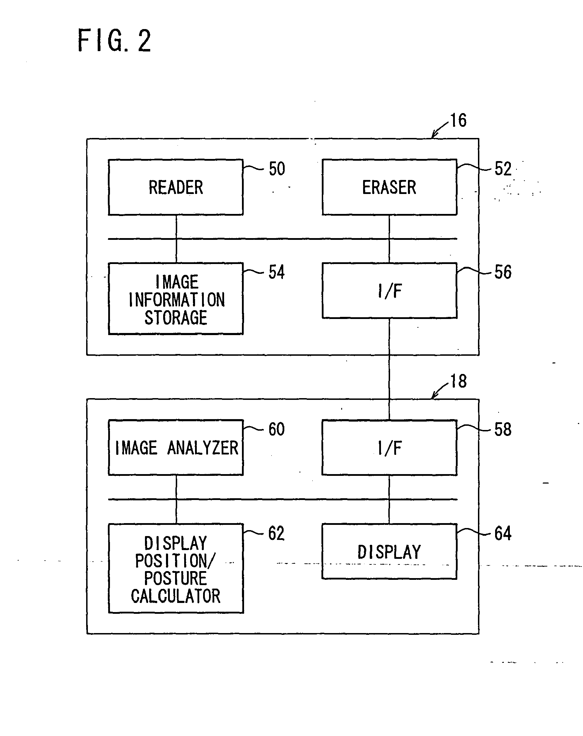

FIG. 2 shows in functional block form the image reading apparatus 16 and the dental radiation image display apparatus 18.

As shown in FIG. 2, the image reading apparatus 16 includes an image information storage 54 for storing radiation image information read by the reader 50, and an interface (I/F) 56 for transmitting radiation image information to the dental radiation image display apparatus 18.

The dental radiation image display apparatus 18 can be implemented by a workstation. The dental radiation image display apparatus 18 includes an interface (I/F) 58 for receiving radiation image information transmitted from the image reading apparatus 16, an image analyzer 60 for analyzing the received radiation image information and extracting feature information of images, a display position/posture calculator 62 for calculating display positions and display postures of segmental images read from the stimulable phosphor panels 28 based on the extracted feature information, and a display 64 for displaying the segmental images in the display positions and display postures that are calculated by the display position/posture calculator 62.

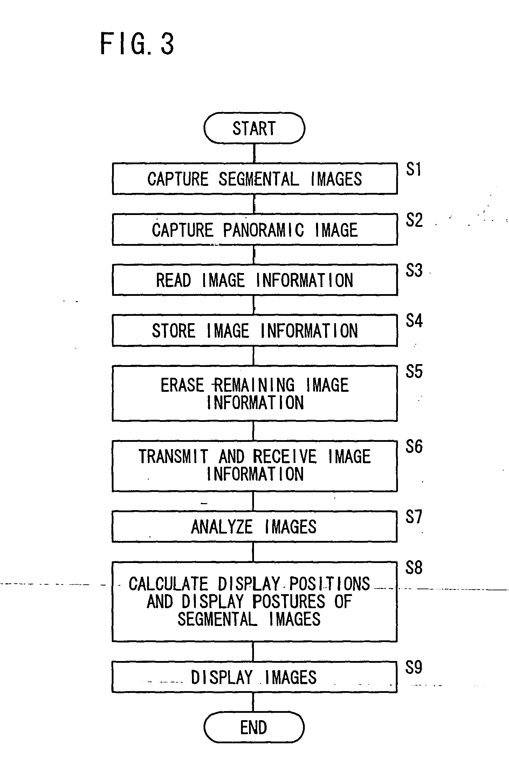

The radiation image information processing system 10 according to the embodiment is basically constructed as described above. Operation and advantages of the radiation image information processing system 10 will be described below with reference to a flowchart shown in FIG. 3.

First, segmental images of teeth of a subject are captured by the segmental image capturing apparatus 12 in step S1. Specifically, a stimulable phosphor panel 28 is positioned at a desired range of teeth to be imaged, and the radiation source housed in the imaging head 26 emits a radiation. The radiation emitted from the imaging head 26 passes through the teeth and is applied to the stimulable phosphor panel 28, recording a segmental image of the teeth on the stimulable phosphor panel 28. If segmental images are captured according the 10-film process, then 10 segmental images of upper and lower teeth are recorded on respective 10 stimulable phosphor panels 28. The 10 stimulable phosphor panels 28 with the corresponding segmental images recorded thereon are mounted in respective positions on the adapter 44, which is then supplied to the image reading apparatus 16.

Then, a panoramic image of all the teeth of the subject is captured by the panoramic image capturing apparatus 14 in step S2. Specifically, the long stimulable phosphor panel 40 is accommodated in the imaging base 42, after which the turning mechanism 34 turns the imaging head 38 and the imaging base 42 around the jaws of the subject, recording a panoramic image on the stimulable phosphor panel 40. The stimulable phosphor panel 40 with the panoramic image recorded thereon is supplied to the image reading apparatus 16. The segmental image capturing apparatus 12 may capture segmental images earlier than the panoramic image capturing apparatus 14 captures a panoramic image, or vise versa.

Thereafter, the image reading apparatus 16 reads the segmental images recorded on the stimulablephosphor panels 28 mounted on the adapter 44 and also reads the panoramic image recorded on the stimulable phosphor panel 40 in step S3.

Specifically, the adapter 44 or the stimulable phosphor panel 40 that is supplied to the image reading apparatus 16 is fed in an auxiliary scanning direction by the feed mechanism 46. While the adapter 44 or the stimulable phosphor panel 40 is being fed in the auxiliary scanning direction, the scanner 48 applies stimulating light as it is being cyclically scanned in a main scanning direction to the stimulable phosphor panels 28 mounted on the adapter 44 or the stimulable phosphor panel 40. Upon exposure to the stimulating light, the stimulable phosphor panels 28 or the stimulable phosphor panel 40 emits stimulated light commensurate with the recorded segmental images or panoramic image. The reader 50 collects the emitted stimulated light and converts the collected stimulated light into an electric signal, thereby reading the segmental images or the panoramic image as radiation image information.

The radiation image information read from the stimulable phosphor panels 28 or the stimulable phosphor panel 40 is once stored in the image information storage 54 in step S4. The stimulable phosphor panels 28 or the stimulable phosphor panel 40 from which the radiation image information has been read by the reader 50 is then exposed to erasing light emitted from the eraser 52. When the stimulable phosphor panels 28 or the stimulable phosphor panel 40 is irradiated with the erasing light, any remaining radiation image information is erased from the stimulable phosphor panels 28 or the stimulable phosphor panel 40 to make the stimulable phosphor panels 28 or the stimulable phosphor panel 40 reusable. The stimulable phosphor panels 28 or the stimulable phosphor panel 40 is ejected from the image reading apparatus 16.

The radiation image information representing the segmental images and the panoramic image, which is stored in the image information storage 54, is transmitted to and received by the dental radiation image display apparatus 18 through the interfaces (I/Fs) 56, 58 in step S6.

Having received the radiation image information, the dental radiation image display apparatus 18 analyzes the radiation image information representing the segmental images in step S7, extracts feature information from the segmental images, and determines the locations of the segmental images.

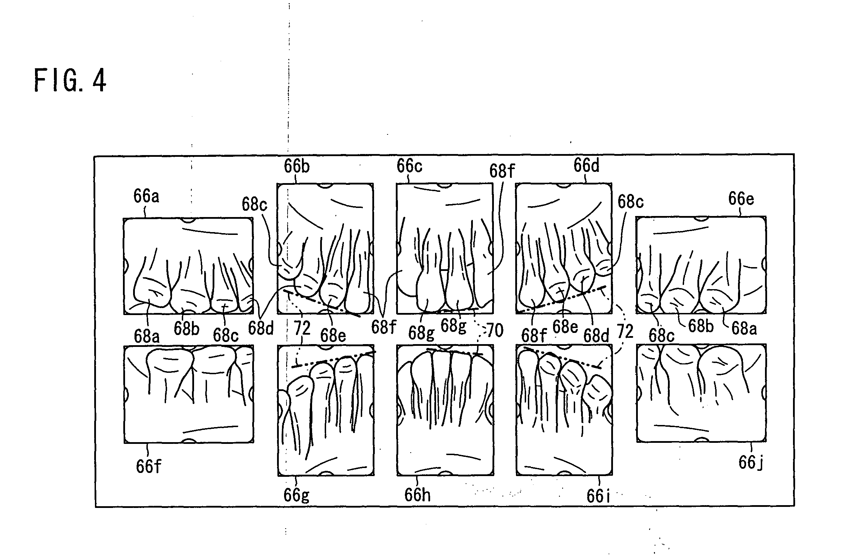

FIG. 4 shows a displayed image including segmental images 66a through 66j captured according to the 10-film process and arranged according to the array of the teeth. The segmental images 66a through 66e represent upper teeth, whereas the segmental images 66f through 66i lower teeth. It is assumed that the teeth are arrayed in a sequence of a second molar 68a, a first molar 68b, a second bicuspid 68c, a first bicuspid 68d, a canine 68e, a lateral incisor 68f, and a central incisor 68g in the order named from the back teeth.

According to the 10-film process, the segmental images 66a, 66e, 66f, 66j including the second molar 68a, the first molar 68b, the second bicuspid 68c, etc. are captured on the stimulable phosphor panels 28 that are oriented horizontally. Therefore, the segmental images 66a, 66e, 66f, 66j and the segmental images 66b, 66c, 66d, 66g, 66h, 66i are sorted out depending on whether they are oriented horizontally or vertically.

From the segmental images 66b, 66c, 66d, 66g, 66h, 66i which are vertically oriented images, the two segmental images 66c, 66h each including the two central incisors 68g which have dental crowns whose tip ends are interconnected by a substantially horizontal straight line (see an additional line 70) are selected. The widths of the central incisors 68g imaged in the selected segmental images 66c, 66h are compared with each other, and the central incisors 68g with the greater widths are judged as upper teeth and the central incisors 68g with the smaller widths as lower teeth. In this manner, the locations of the segmental images 66c, 66h are determined.

Then, of the segmental images 66b, 66d, 66g, 66i which are vertically oriented images, the angles of inclination of straight lines (see additional lines 72) interconnecting the tip ends of the dental crowns are calculated, and these segmental images are determined as indicating left and right teeth based on the calculated angles of inclination. Then, the lengths of dental roots of the longest teeth (the canine 68e) are compared with each other, and the teeth of the longer dental roots are judged as upper teeth and those of the shorter dental roots as lower teeth. In this manner, the locations of the segmental images 66b, 66d, 66g, 66i are determined.

Then, of the segmental images 66a, 66e, 66f, 66j which are horizontally oriented images, the shapes of the jawbones are compared with each other to determine upper segmental images 66a, 66e and lower segmental images 66f, 66j. The widths of the second bicuspid 68c and the first molar 68b adjacent thereto are compared with each other. The wider tooth is judged as the first molar 68b. The locations of the left and right segmental images 66a, 66e, 66f, 66j are determined from the angle of inclination of the first molar 68b.

The locations of the segmental images 66a through 66j may be determined based on not only the feature information such as the direction in which they are oriented, the widths and lengths of the teeth, etc., but also therapeutic information of the teeth which has been acquired in advance.

After the locations of the segmental images 66a through 66j have been determined based on the feature information of the teeth, the display position/posture calculator 62 calculates display positions and display postures on the display 64 of the segmental images 66a through 66j whose locations have been determined by the image analyzer 60 in step S8.

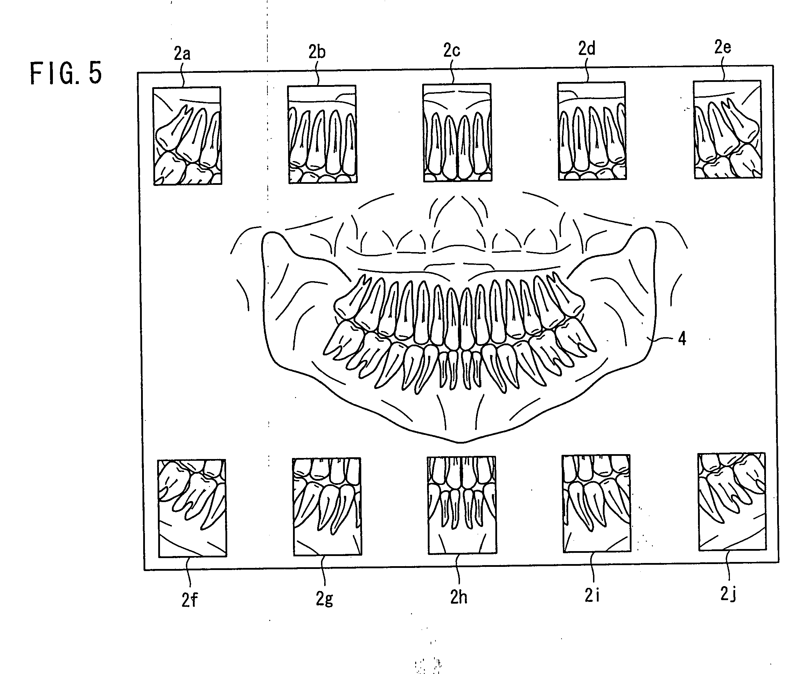

The display 64 displays the segmental images 66a through 66j in the determined locations based on the display positions and display postures that have been calculated, as shown in FIG. 4, in step S9. The segmental images 66a, 66e, 66f, 66j_which are horizontally oriented images are displayed in horizontal display postures, and the segmental images 66b, 66c, 66d, 66g, 66h, 66i which are vertically oriented images are displayed in vertical display postures. As with the related art shown in FIG. 5, the panoramic image read from the stimulable phosphor panel 40 is displayed in addition to the segmental images 66a through 66j for allowing the association between the positions of the displayed teeth to be grasped more clearly.

In the above embodiment, the locations of the segmental images 66a through 66j are determined by extracting the feature information of the segmental images 66a through 66j. However, the image analyzer 60 (pattern matching means) may compare the segmental images 66a through 66j with the panoramic image, and may identify regions of the panoramic image which correspond to the segmental images 66a through 66j according to a pattern matching process to determine the locations of the segmental images 66a through 66j.

## Claims

1. A dental radiation image display apparatus (18) for displaying a plurality of segmental images, which are set in respective predetermined positions, of teeth of a subject which are imaged by a radiation image capturing apparatus (12), comprising:
image analyzing means (60) for analyzing the segmental images and extracting feature information from the segmental images;
display position calculating means (62) for calculating respective display positions of the segmental images depending on the locations of teeth based on the extracted feature information; and
display means (64) for displaying the segmental images in the calculated display positions.

2. A dental radiation image display apparatus according to claim 1, wherein said image analyzing means (60) extracts directions in which said segmental images are oriented on recording mediums as said feature information.

3. A dental radiation image display apparatus according to claim 1, wherein said image analyzing means (60) extracts angles of inclination of straight lines interconnecting the tip ends of the teeth in the segmental images as said feature information.

4. A dental radiation image display apparatus according to claim 1, wherein said image analyzing means (60) extracts widths of the teeth in the segmental images as said feature information.

5. A dental radiation image display apparatus according to claim 1, wherein said image analyzing means (60) extracts lengths of the teeth in the segmental images as said feature information.

6. A dental radiation image display apparatus according to claim 1, wherein said image analyzing means (60) extracts therapeutic information of the teeth as said feature information.

7. A dental radiation image display apparatus according to claim 1, further comprising
display posture calculating means (62) for calculating respective display postures of the segmental images,
wherein said display means (64) displays the segmental images in the calculated display postures.

8. A dental radiation image display apparatus (18) for displaying a plurality of segmental images, which are set in respective predetermined positions, of teeth of a subject which are imaged by a radiation image capturing apparatus (12), comprising:
pattern matching means (60) for comparing the segmental images with a panoramic image of the teeth and identifying regions on the panoramic image which correspond to the segmental images;
display position calculating means (62) for calculating respective display positions of the segmental images depending on the locations of teeth based on the identified regions on the panoramic image; and
display means (64) for displaying the segmental images in the calculated display positions.

9. A dental radiation image display apparatus according to claim 8, further comprising
a panoramic image capturing apparatus (14) for capturing said panoramic image.
